# EUROPEAN PATENT APPLICATION

(11) **EP 3 960 113 A1**
(43) Date of publication of application: **02.03.2022**
(21) Application number: 21425040.9
(22) Date of filing: 20.08.2021
(51) Int. Cl.: A61B 90/00, A61B 17/12

(54) **MEDICAL DEVICE FOR MECHANICAL HEMOSTASIS OF BLOOD VESSELS IN VIDEO ASSISTED EMERGENCY SURGERY**

(30) Priority: 25.08.2020 IT 202000020383
(71) Applicant: Biodismed s.r.l, 00071 Pomezia (RM) (IT)
(72) Inventor: Maggiori, Roberto, 00071 Pomezia (RM) (IT)

(57) **Abstract**

The present invention relates to a portable medical device suitable for video-assisted emergency surgery, equipped with all the components necessary for the mechanical hemostasis of the blood vessels of patients who have suffered serious injuries and who require immediate surgery directly on the site of the trauma.

This device allows the application of metal clips to be carried out simultaneously providing both the aspiration of the blood effusion facilitating the emptying of the surgical or traumatic breach, and to deliver physiological solution useful for washing the operating field.

## Description

The present invention is generally applicable in the medical-health field, in the field of invasive surgical devices, and more precisely it relates to a portable medical device, useful for the mechanical hemostasis of blood vessels, particularly suitable for operating outside hospitals, for patients affected by severe trauma who require surgery directly on site and / or during transport to the hospital.

### BACKGROUND ART

Currently, as regards the operation of mechanical hemostasis of the blood vessels, mostly manually operated fork-type metal clip applicators are used, however, it frequently happens that the blood vessel to be occluded by means of the application of the clip, is affected by hemorrhage severe, and for this circumstance the bleeding of the vessel can cause a blood effusion inside the surgical or traumatic breach, which constitutes an obstacle for the surgeon who cannot easily identify the end of the vessel to be occluded, and for this reason it is necessary the help of a second health care professional who has the task of using a fluid aspirator to remove this effusion.

In fact, this type of operation is easy within hospitals where it is possible to employ a medical team that performs every single task, but when the need arises to intervene directly on the place where the patient has been affected by trauma, it is of vital importance for the surgeon to carry out this operation independently.

For this reason, the device object of the invention is equipped with all the components necessary for mechanical hemostasis, easily transportable inside a single openable container equipped with a handle, comprising an aspirator for fluids, a pump for dispensing the physiological solution, an interchangeable tank for the collection of fluids, a tank for the supply of physiological solution to be dispensed and a general control panel equipped with a second display suitable for assisted viewing of the operating image, entirely powered by rechargeable batteries.

### SUMMARY OF INVENTION

A primary purpose of the present invention is to promptly operate in emergency situations such as for example accidents involving means of transport or natural disasters, where it is necessary to intervene directly on the place where the patient has suffered the trauma, having a complete instrumentation which can be used autonomously by the surgeon and that he is able to stop the bleeding of the affected blood vessels in the shortest possible time.

Another purpose of the present invention is that of being able to apply the metal clip for the mechanical hemostasis of the blood vessel being able to simultaneously provide both the aspiration of the blood effusion facilitating the emptying of the surgical or traumatic breach from fluids, and to deliver physiological solution useful for washing of the blood vessel to be treated, as well as useful for cleaning the optics of the self-illuminated micro camera placed inside the barrel of the device.

Another purpose of the present invention is to aspire the bleeding vessel inside the cannula located at the center of the device barrel, in order to aspire the blood into a tank placed in the transportable container, as well as to position and fix the blood vessel to be treated, inside the circular section metal clip, proceeding with the crimping operation, which consists in tightening the circular section clip around the bleeding vessel, causing mechanical occlusion through the use of the device.

Another purpose of the present invention is to operate quickly in laparoscopy by means of assisted vision, inserting the prolonged barrel of the device in the surgical opening, using a self-illuminated micro-camera placed near the crimping jaws of the metal clips, showing the operating image both on the display incorporated in the device itself and on the remote display on the general control panel of the transportable container.

Another purpose of the present invention is to make everything necessary for the hemostasis operation easily transportable, through the use of a single container equipped with a handle, which comprises the clip applicator device, the fluid aspirator, the pump for the delivery of physiological solution and the system for the acquisition, vision and recording of images in video-surgery.

The invention therefore allows to achieve the following advantages:
Rescue the patient at risk of bleeding in the same place where the trauma occurred, operating even before transporting him to an equipped hospital that takes longer.

Significantly reduce the intervention times, thus increasing the chances of survival of the traumatized patient at risk of exsanguination.

Significantly reduce the overall dimensions of the transportable medical equipment, to facilitate the mechanical hemostasis operation independently, through the use of the device.

Have an "all in one" device that is a single compact and ready to use machine body, capable of simultaneously carrying out all the operations necessary for the mechanical hemostasis of blood vessels, including the application of the clips, the aspiration of fluids and the supply of physiological solution always using the same device.

Have assisted vision and then operate in video surgery through the use of the self-illuminated micro camera, also suitable for recording surgical images, as well as for use in telemedicine modes.

### BRIEF DESCRIPTION OF THE DRAWINGS

The device and the method of the invention are susceptible of numerous modifications and variations, all of which fall within the inventive concept expressed in the attached claims.

All the details can be replaced by other technically equivalent elements, and the materials can be different according to the requirements, without departing from the scope of protection of the present invention.

Although the device has been described with particular reference to the attached figures, the reference numbers used in the description and in the claims are used to improve the intelligence of the invention and do not constitute any limitation to the scope of protection claimed:
- **Fig. 1** represents an isometric view of the device on two projections, in an assembled configuration;
- **Fig. 2** is an exploded isometric view of the device;
- **Figs. 3** and **4** show a side view of the device comprising respectively the enlarged views of the details A and B;
- **Fig. 5** represents a front view of the device comprising an enlarged view of detail C;
- **Fig. 6** represents an exploded axonometric view of the device comprising the enlarged detail view D;
- **Fig. 7** represents a sectional view of the device comprising the enlarged detail E;
- **Fig. 8** represents an axonometric view of the device in operational configuration connected to the components located inside the transportable container.
- **Fig. 9** represents a plan view of the metal clip before and after mechanical crimping through the use of the device.

### DESCRIPTION OF A PREFERRED EMBODIMENT

The invention is susceptible of numerous modifications and variations, all falling within the inventive concept expressed in the attached claims.

All the details can be replaced by other technically equivalent elements, and the materials can be different according to the requirements, without departing from the scope of protection of the present invention.

Although, the invention has been described with particular reference to the attached figures, the reference numbers used in the description and in the claims are used to improve the intelligence of the invention and do not constitute any limitation to the scope of protection claimed.

Further characteristics and advantages of the invention will become more evident in the light of the detailed description of a preferred but not exclusive embodiment of the device according to the invention, illustrated by way of nonlimiting example, with the aid of the attached drawing tables where 1 globally indicates a medical device for mechanical hemostasis of blood vessels in video-assisted emergency surgery according to the invention, in which:

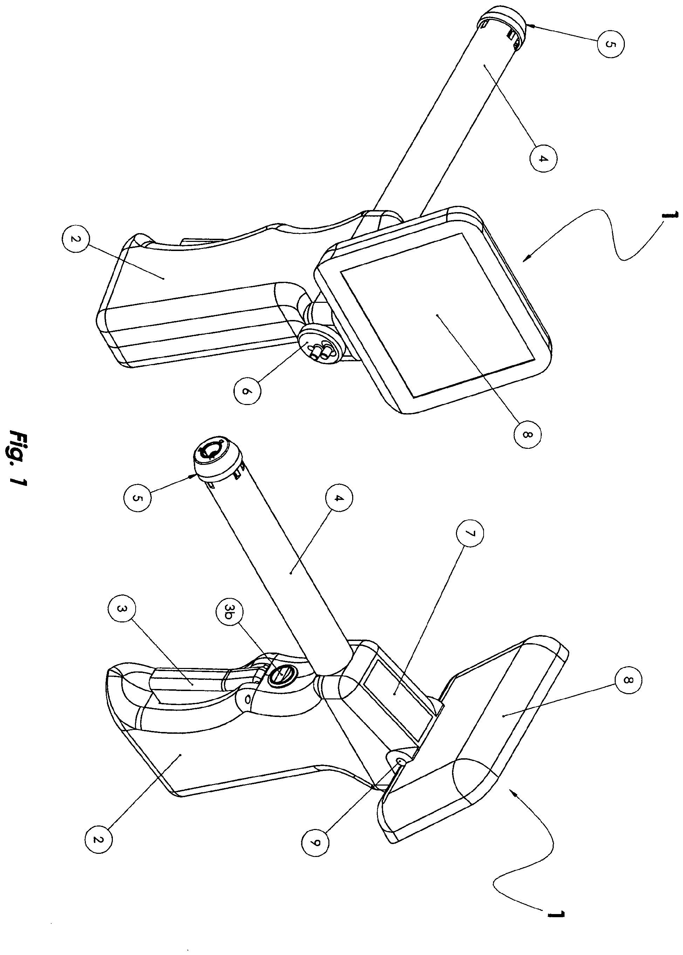

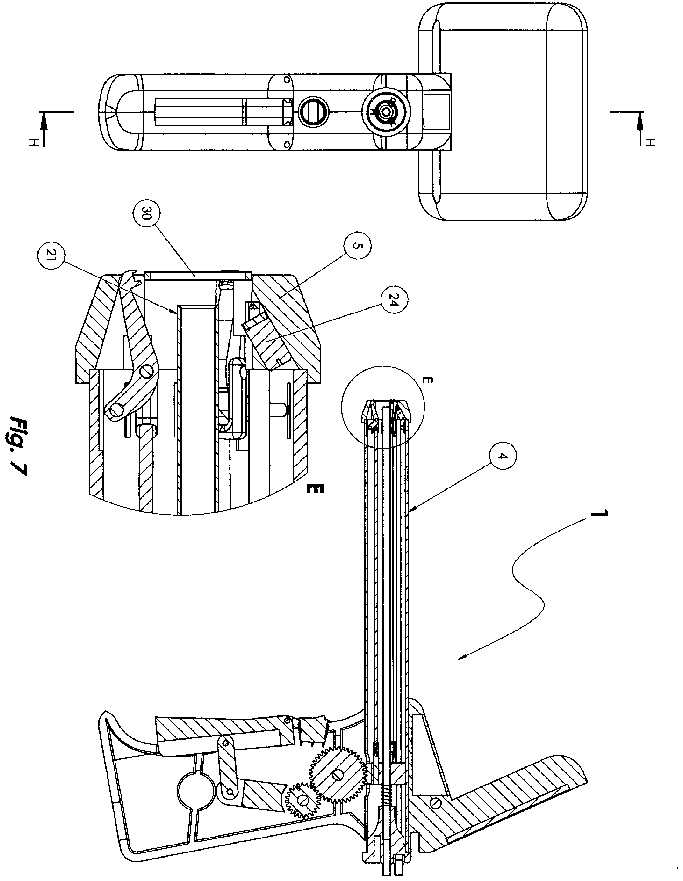

**Fig. 1** shows a device 1 comprising a handle 2 equipped with an operating lever 3 and a rocker switch 3b, which in the upper part centrally houses a barrel 4 which ends at a first end with a front cap 5 which houses a self-illuminated micro-camera 24 (see fig. 7 detail E), while in the opposite end it ends with a rear cap 6, in which a body 7 is connected on the top of said handle 2 which houses an LCD display 8 which can be oriented by means of a pin 9 which acts joint between the coupling of said body 7 and the LCD display 8.

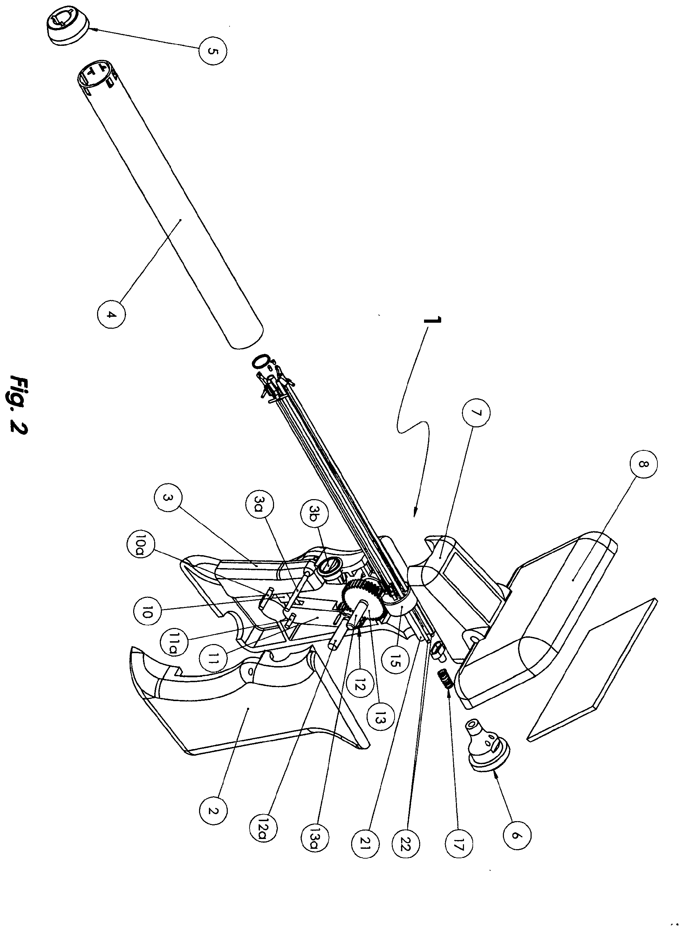

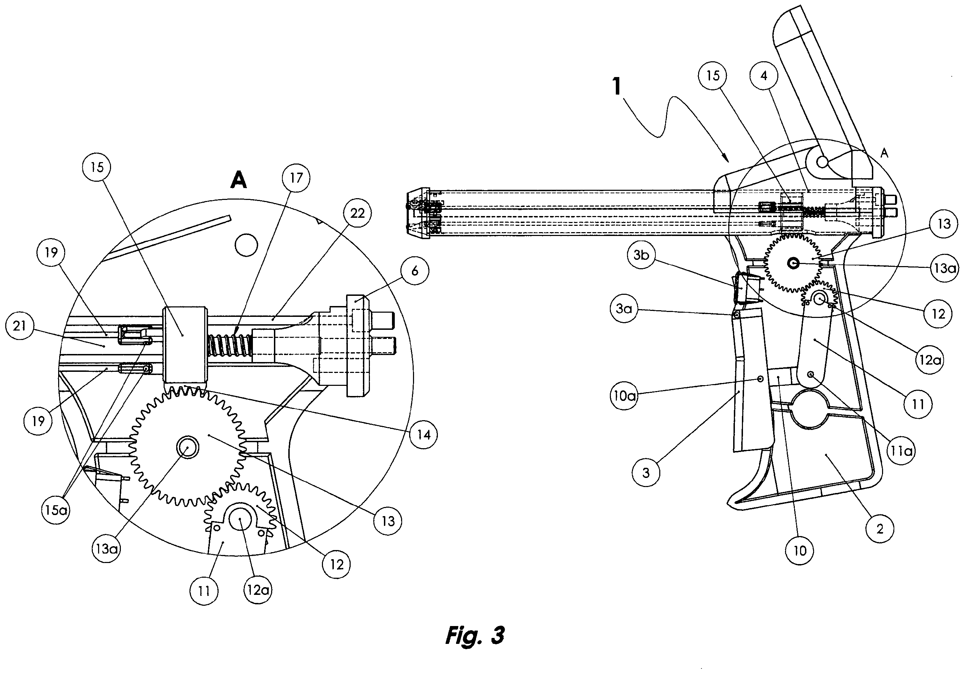

**Fig. 2** and **3** show the mechanism placed inside the device 1 in which the upper end of said operating lever 3 is hinged to said handle 2 by means of a first pin 3a, while the intermediate region of said operating lever 3 is hinged to a first return arm 10 by means of a second pin 10a, in which said return arm 10 is connected to a second return arm 11 by means of a third pin 11a, in which said return arm 11 is integral with a toothed wheel 12 pivoted on said handle 2 by means of the pin 12a, in which said toothed wheel 12 is coupled to a second toothed wheel 13 hinged on said handle 2 by means of the pin 13a, in which said toothed wheel 13 is coupled to a rack 14 integral with a movable bushing 15 which, as better seen in detail A of fig. 3, slides axially along the internal seat of the barrel 4, in which said movable bushing 15 is placed in contrast with the rear cap 6 by means of a spring 17 which slides axially around the central suction cannula 21, in which said spring 17 a following the manual actuation determined by the pressure exerted by the surgeon on the actuation lever 3, it determines a counterthrust necessary for the return to the initial position of the crimping mechanism.

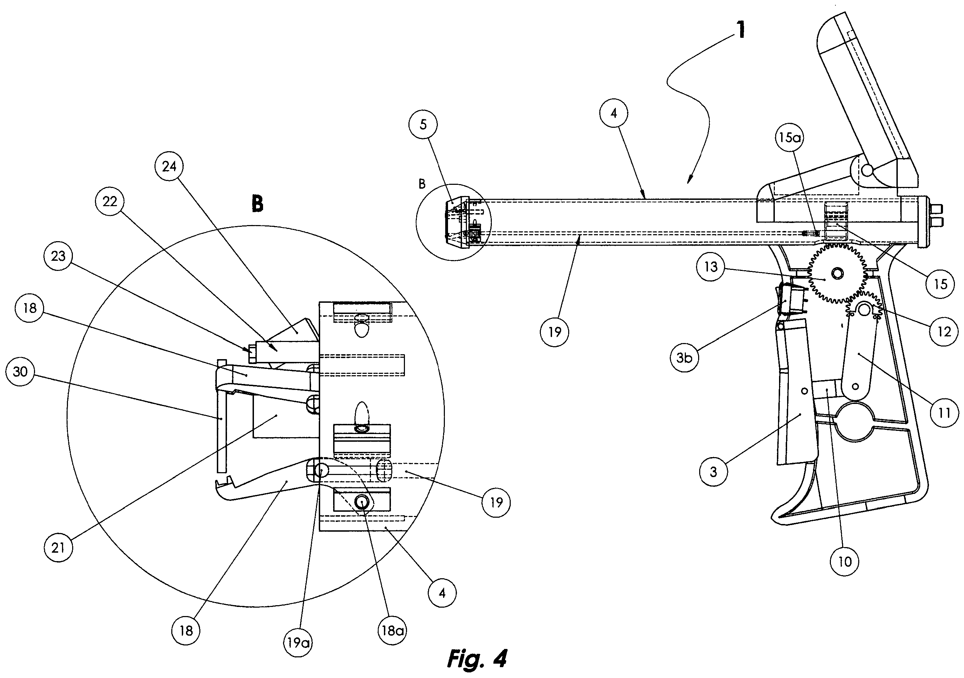

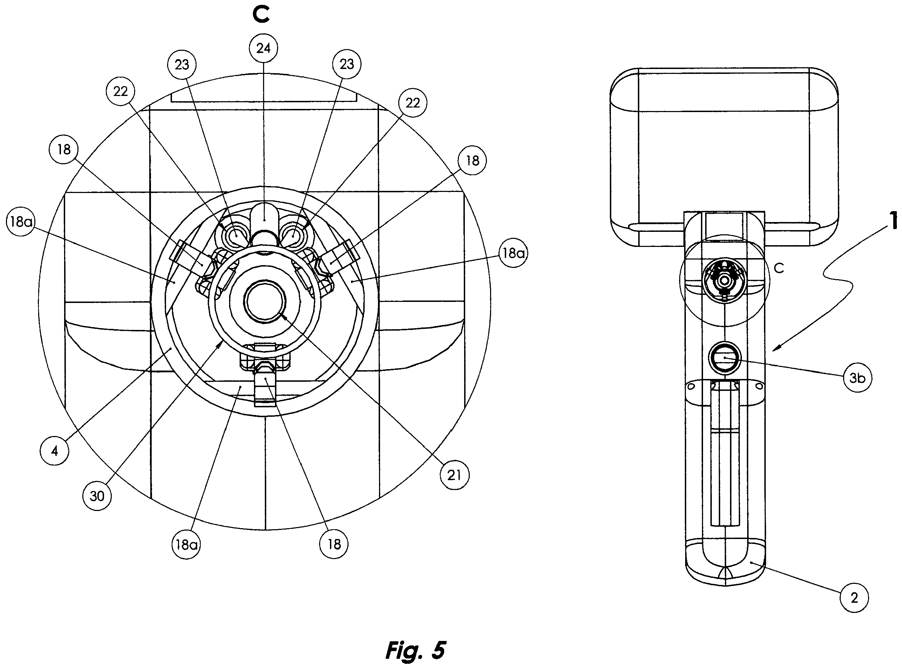

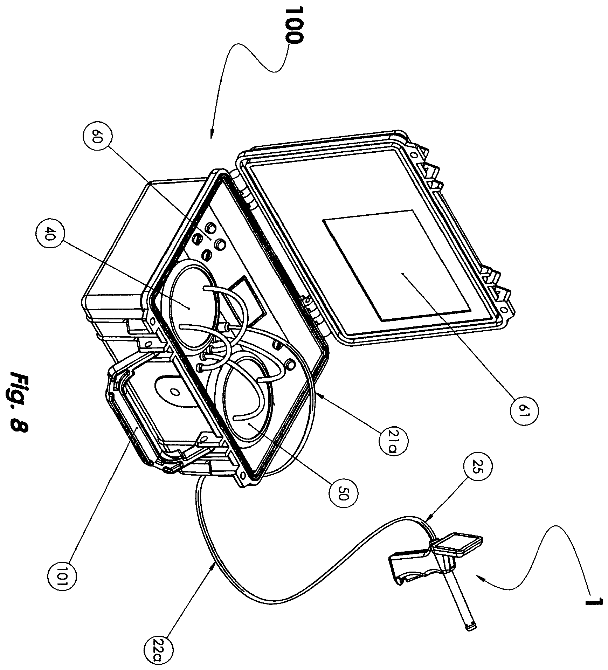

**Fig. 4** shows detail B concerning the end of the barrel 4 which houses crimping jaws 18 hinged on said barrel 4 by means of the pin 18a (see fig. 5), in which fork tie rods 19 having one end connected to said jaws crimping 18 through the pin 19a, the opposite end connected to the movable bushing 15 through the pin 15a (see fig. 3) which, following the aforementioned manual operation by the surgeon through the operating lever 3, said fork tie rods 19 exert a traction force on said jaws 18, causing the concentric closure of said crimping jaws 18 which allow crimping of the circular section metal clip 30 around the blood vessel to be occluded, determining a characteristic geometric shape of said clip 30 (see fig. 8).

**Fig. 5** shows the front detail C of the three crimping jaws 18 in the open position at rest, which hold the circular section metal clip 30 in position before the crimping / closing phase, and in particular the central suction tube 21 is visible. it is connected by means of a flexible tube 21a to the fluid aspirator located inside the transportable container 100 (see fig. 8), which can be activated by means of the rocker switch 3b located on the handle 2 of the device 1 (see fig. 1), in which said central suction cannula 21 is suitable both for sucking and fixing the blood vessel inside it, and for sucking up the blood effusion, ensuring at the same time the maximum precision of positioning of the circular section metal clip 30 around the blood vessel to occlude.

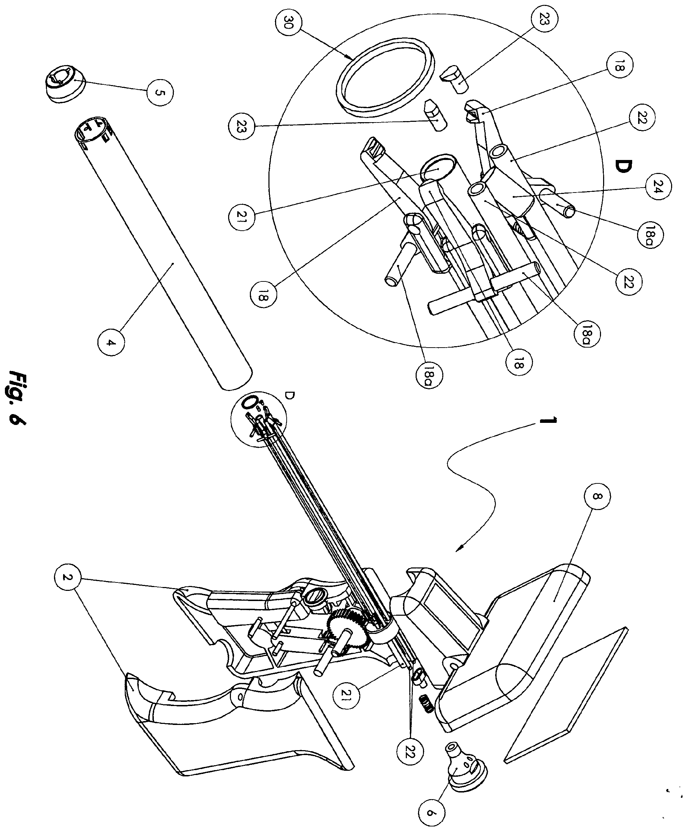

**Fig. 6** shows in axonometric projection the detail D concerning the complex of the crimping jaws 18, comprising the aforementioned central suction tube 21 and the conduits saline solution delivery ducts 22 having a first end coupled to double jet nozzles 23 (see fig. 5), while at the opposite end they are connected by a flexible tube 22a to the physiological solution dispensing pump located inside the container transportable 100 (see fig. 8), operable by means of the rocker switch 3b located on the handle 2 of the device 1 (see fig. 1), in which said physiological solution delivery ducts 22 through said double jet nozzles 23, they are suitable both for washing the end of the blood vessel to be treated and for washing the optics of the self-illuminated micro camera 24 housed in the upper part of the front cap 5 located at the end of the barrel 4 (see fig. 4 and 5), in which said self-illuminated micro-camera 24 simultaneously sends the video images both to the LCD display 8 incorporated in the device 1, and to the LCD display 61 located inside the container transportable tore 100, through the wired connection 25 which also performs the power supply (see fig. 8), in which said self-illuminated micro-camera 24 is suitable for video-assisted exploration of the patient's surgical or traumatic breach, with the aim of identifying the bleeding vessel to be occluded by means of the application of the circular section clips 30 through the use of the device 1.

**Fig. 7** shows detail E concerning the section of the front cap 5, in which the housing obtained for the self-illuminated micro camera 24 pointing in the area is visible in which the blood vessel is sucked into the central suction tube 21 before applying the circular section metal clip 30.

**Fig. 8** is an overall view comprising the device 1 in operating configuration connected to the components included inside the portable container 100 equipped with a handle 101, which contains inside it a fluid aspirator not shown in the figure, connected to a fluid collection tank 40, a physiological solution dispensing pump not shown in the figure, connected to a physiological solution tank 50, a power supply stage not shown in the figure, suitable for power supply supplied by rechargeable batteries, a general control panel 60 and a display LCD 61.

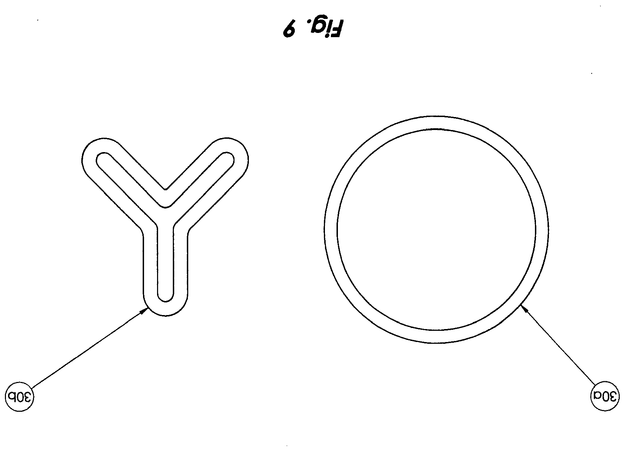

**Fig. 9** shows the clip 30 respectively in the initial open shape 30a, and in the closed crimped shape 30b.

## Claims

1. Medical device for mechanical hemostasis of blood vessels in video assisted emergency surgery (1), **comprising** a handle (2) equipped with an operating lever (3) and a rocker switch (3b), in which in the the upper part of said handle (2) is centrally housed a barrel (4) comprising inside three crimping jaws (18), three tie fork rods (19), a central suction pipe (21), two physiological saline solution pipes (22), two double jet nozzles (23) and a circular metal clip (30), in which said barrel (4) has a first free end terminates with a cone end cap (5) which houses a self-illuminated micro camera (24), at the opposite end terminates with a rear end cap (6), in which a body (7) is connected to the top of said handle (2) which houses an LCD display (8) which can be oriented by means of a pin (9) which acts as a joint between the coupling of said body (7) and said LCD display (8), **characterized in that** said operating lever (3) is pivoted on to said handle (2) by means of a first pin (3a), in which the central portion of said operating lever (3) is pivoted on to a first transmission arm (10) by means of a second pin (10a), in which said transmission arm (10) is connected to a second transmission arm (11) by means of a third pin (11a), in which said transmission arm (11) is integral with a toothed wheel (12), in which said toothed wheel (12) is coupled to a second toothed wheel (13) hinged on said handle (2) by means of the pin (13a), in which said toothed wheel (13) is coupled to a toothed rack (14) integral with a sliding bush (15) which slides axially along the internal seat of the barrel (4), in which said sliding bush (15) is placed in contrast with the rear end cap (6) by means of a spring (17) which slides axially around the central suction pipe (21), in which said spring (17) following the manual operation determined by the pressure on the operating lever (3) exerted by the surgeon, determines a counterthrust necessary to the return in the initial position of the crimping mechanism of the device (1), in which said barrel (4) houses said crimping jaws (18) hinged on said barrel (4) by means of the pin (18a), in which three tie fork rods (19) having one end connected to said crimping jaws (18) by means of the pin (19a), the opposite end connected to the said sliding bush (15) by means of the pin (15a) which following said manual operation by the surgeon through the operating lever (3), said tie fork rods (19) exert a traction force on said crimping jaws (18), causing the concentric closure of said crimping jaws (18) which allow to crimp the circular metal clip (30) around the blood vessel to be occluded.

2. Medical device for mechanical hemostasis of blood vessels in video-assisted emergency surgery (1) according to claim 1, **characterized in that** said central suction pipe (21) is suitable both to suction and fixing the vessel to be treated, and to aspirate the blood effusion.

3. Medical device for mechanical hemostasis of blood vessels in video-assisted emergency surgery (1) according to claim 2, **characterized in that** said central suction pipe (21) is connected by means of a flexible tube (21a) to the liquid aspirator equipped with a tank (40) placed inside the transportable case (100), which can be activated by means of the rocker switch (3b) located on the handle (2) of the device (1).

4. Medical device for mechanical hemostasis of blood vessels in video-assisted emergency surgery (1) according to claim 1, **characterized in that** said physiological saline solution pipes (22) have a first end coupled to double jet nozzles (23), and at the opposite end they are connected by means of a flexible tube (22a) to the physiological solution pump equipped with a tank (50) located inside the transportable case (100), which can be operated by means of the rocker switch (3b) on the handle (2) of the device (1).

5. Medical device for mechanical hemostasis of blood vessels in video-assisted emergency surgery (1) according to claim 1, **characterized in that** said cone end cap (5) is located at the end of the barrel (4), and houses a self-illuminating micro-camera (24) suitable for video-assisted exploration of the patient's surgical or traumatic incision, with the aim of identifying the bleeding vessel to be occluded by applying the circular metal clip (30) using the device (1).

6. Medical device for mechanical hemostasis of blood vessels in video-assisted emergency surgery (1) according to claim 1, **characterized in that** said physiological saline solution pipes (22) coupled to said double jet nozzles (23) are suitable both for washing the extremity of the blood vessel to be treated and for washing the optics of the self-illuminating micro camera (24) housed in the upper part of the cone end cap (5) located at the end of the barrel (4).

7. Medical device for the mechanical hemostasis of blood vessels in video-assisted emergency surgery (1) according to claim 5, **characterized in that** said self-illuminating micro camera (24) transmits simultaneously the video images both to the LCD display (8) incorporated in the device (1) and to the LCD display (61) placed inside the transportable case (100) through the wired connection (25) which also suitable to the power source.

8. Medical device for the mechanical hemostasis of blood vessels in video-assisted emergency surgery (1) according to any previous claims, **characterized in that** said transportable case (100) contains inside, a liquid aspirator equipped with a tank (40), a physiological solution pump equipped with a tank (50), a power stage suitable for the electrical supply by rechargeable batteries, a general control panel (60) and an LCD display (61).
